(19) Europäisches Patentamt
European Patent Office
Office européen des brevets

(11) Veröffentlichungsnummer: **0 034 367**
**B1**

(12) # EUROPÄISCHE PATENTSCHRIFT

(45) Veröffentlichungstag der Patentschrift: **29.06.83**

(51) Int. Cl.³: **C 07 J 41/00**

(21) Anmeldenummer: **81101118.8**

(22) Anmeldetag: **17.02.81**

(54) **Verfahren zur hydrolytischen Spaltung von Steroid-20-Carbamaten.**

(30) Priorität: **19.02.80 US 122321**

(43) Veröffentlichungstag der Anmeldung: **26.08.81 Patentblatt 81/34**

(45) Bekanntmachung des Hinweises auf die Patenterteilung: **29.06.83 Patentblatt 83/26**

(84) Benannte Vertragsstaaten: **AT BE CH DE FR GB IT LI LU NL SE**

(56) Entgegenhaltungen:

**HELVETICA CHIMICA ACTA, Band 28, Nr. 2, 15. März 1945, Seiten 334, 336, Basel, CH.,
P. RUGGLI et al.: «Indolin aus o-amino-phenäthylamin»**

(73) Patentinhaber: **HENKEL CORPORATION, 4620 West 77th Street, Minneapolis Minnesota 55435 (US)**

(72) Erfinder: **Krbechek, Leroy O., 2041 Orkla Drive, Minneapolis Minnesota 55427 (US)**

(74) Vertreter: **Fues, Johann Friedrich, Dr. et al, Deichmannhaus am Hauptbahnhof, D-5000 Köln 1 (DE)**

EP 0 034 367 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur hydrolytischen Spaltung von Steroid-20-Carbamaten

Die Erfindung betrifft die Umwandlung bestimmter Steroid-20-Carbamate unter selektiver hydrolytischer Spaltung der Carbamatgruppe in 20-Stellung und beschreibt damit eine wichtige Stufe auf dem Weg der Gewinnung von Progesteron aus Ausgangsmaterialien, die im technischen Massstab zur Verfügung stehen und sich letztlich von Steroidverbindungen pflanzlichen und/oder tierischen Ursprungs ableiten.

In der Europäischen Patentanmeldung 004 913 ist u.a. ein Verfahren zur Herstellung von 3-Oxo--pregna-4-en-20-carbonsäure($\triangle^4$-BNC) durch mikrobiellen Seitenkettenabbau an 17-C-Seitenkettensteroidsubstraten beschrieben. In vergleichbarer Weise kann gemäss der Lehre der US-PS 3 994 933 eine verwandte Verbindung erhalten werden, nämlich die 3-Oxo-pregna-4,17(20)-dien-20-carbonsäure($\triangle^{4,17(20)}$-BNC).

Zur Umwandlung dieser Ausgangsmaterialien in Progesteron (3,20-Dioxo-pregn-4-en) bedarf es des Abbaus der in 20-Stellung substituierten Carboxylgruppe und der Umwandlung von C-20 des Steroidgerüsts in die Oxogruppe.

Es ist eine Reihe von Möglichkeiten entwickelt worden, den partiellen Abbau von $\triangle^4$-BNC und/oder $\triangle^{4,17}$-BNC derart zu führen, dass als Endprodukt dieses partiellen Abbaus die entsprechenden Steroid-20-Carbamate anfallen, d.h. Steroidverbindungen, die in 20-Stellung die Carbamatgruppe -NHCOOR aufweisen. Einzelheiten hierzu sind beispielsweise in den parallelen Europäischen Patentanmeldungen 81 101 119.6 (34 368) («Verfahren zur Umwandlung von Steroid-20-Carbonsäureamiden in die entsprechenden Steroid-20-Carbamate», U.S.-Priorität vom 19. Februar 1980, Aktenzeichen: 122 395,
81 101 117.0 (34 366) («Verfahren zur Herstellung von Steroid-20-Carbamatverbindungen», U.S.Priorität vom 19. Februar 1980, Aktenzeichen: 122 398,
81 101 114.7 (34 364) («Neue 20-substituierte 3-Oxo-pregna-4-en-steroidverbindungen und Verfahren zu ihrer Herstellung», U.S.Priorität vom 19. Februar 1980, Aktenzeichen: 122 397
der gleichen Anmelderin beschrieben.

Formelmässig lässt sich der Sachverhalt wie folgt verdeutlichen: $\triangle^4$-BNC bzw. $\triangle^{4,17}$-BNC entsprechen den Strukturformeln A bzw. B mit der Massgabe, dass X die Carboxylgruppe -COOH darstellt.

A

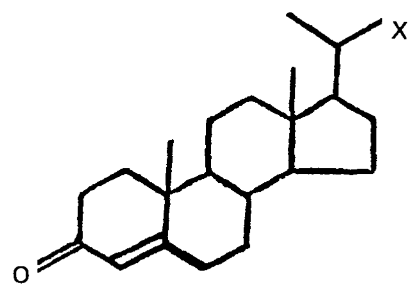

B

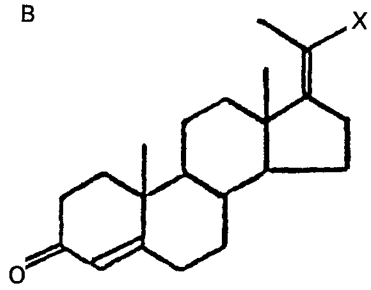

In den ensprechenden Steroid-20-Carbamatverbindungen hat X die Bedeutung von -NHCOOR. Man erhält die Carbamate aus den Säuren auf einem mehrstufigen Weg, der beispielsweise wie folgt verlaufen kann: Zunächst wird das Säureausgangsmaterial (X = -COOH) durch Umsetzung mit einem Halogenierungsmittel, insbesondere Thionylchlorid, zum Säurehalogenid bzw. Säurechlorid umgewandelt. Durch Behandlung des Säurechlorids mit Ammoniak bzw. Ammoniumhydroxid kann das entsprechende Säureamid gebildet werden. Durch N-Bromierung dieses Säureamids und Behandlung des so gebildeten Bromamids mit einer starken Base in Gegenwart einwertiger Alkohole bzw. durch Behandlung des Bromamids mit der starken Base und anschliessende Umsetzung des intermediär gebildeten Reaktionsproduktes mit den einwertigen Alkoholen werden die Steroid-20-Carbamate ausgebildet. Unter Anlehnung an die Formelbilder A und B gilt: Die jeweilige BNC-Verbindung (X = -COOH) wird zum Säurechlorid (X = -COCl) und dann zum Säureamid (X = -$CONH_2$) umgewandelt.

Über die Bildung des Bromamids (X = -CONHBr) kann letztlich das Steroid-20-Carbamat (X = -NHCOOR) erhalten werden. Eine andere Möglichkeit des Überganges von der 20-Carboxamidoverbindung zum Carbamat liegt in der Umsetzung der erstgenannten Steroidverbindung mit Bleitetraacetat unter Ausbildung des entsprechenden Isocyanats und dessen Reaktion mit Alkohol zum Carbamat. Einzelheiten zu diesen Reaktionsstufen finden sich beispielsweise in der älteren Europäischen Patentanmeldung 81 100 145.2 sowie in den parallelen Europäischen Patentanmeldungen 81 101 114.7 (34 364) («Neue 20-substituierte 3-Oxo-pregna-4-ensteroidverbindungen und Verfahren zu ihrer Herstellung», U.S.Priorität vom 19. Februar 1980, Aktenzeichen: 122 397, Case 4168) 81 101 119.6 (34 368) («Verfahren zur Umwandlung von Steroid--20-Carbonsäureamiden in die entsprechenden Steroid-20-Carbamate», U.S.Priorität vom 19. Februar 1980, Aktenzeichen: 122 395, Case 4166) 81 101 117.0 (34 366) («Verfahren zur Herstellung von Steroid-20-Carbamatverbindungen», U.S.Priorität vom 19. Februar 1980, Aktenzeichen: 122 398, Case 4174) der gleichen Anmelderin.

Die vorliegende Erfindung geht von der Aufgabe aus, die den Formelbildern A und B entsprechenden Carbamatverbindungen (X = -NHCOOR) einer selektiven Spaltung der Carbamatgruppe in 20-Stellung zu unterwerfen.

Gegenstand der vorliegenden Erfindung ist dementsprechend ein Verfahren zur selektiven hydrolytischen Spaltung von Steroid-20-Carbamaten aus der Gruppe 3-Oxo-pregn-4-en-20-carbamat und/oder 3-Oxo-pregna-4,17(20)-dien-carbamat, wobei dieses Verfahren dadurch gekennzeichnet ist, dass man zur Spaltung der Carbamatgruppe in 20-Stellung das Ausgangsmaterial der Behandlung mit einer Halogenwasserstoffsäure unterwirft. Bevorzugt wird mit wässrig-konzentrierten Halogenwasserstoffsäuren gearbeitet, wobei insbesondere konzentrierte Salzsäure ggf. aber auch wässrig-konzentrierte Bromwasserstoffsäure Verwendung finden.

In der bevorzugten Ausführungsform des erfindungsgemässen Verfahrens wird zur sauren hydrolytischen Spaltung eine wässrig-konzentrierte Halogenwasserstoffsäure eingesetzt, die zusätzlich mit dem entsprechenden gasförmigen Halogenwasserstoff gesättigt worden ist. Das bevorzugte Hydrolysemedium gemäss der Erfindung ist konzentrierte Salzsäure, die mit gasförmigem HCl gesättigt worden ist.

Als Alkoholkomponente in den erfindungsgemäss eingesetzten Carbamaten können grundsätzlich beliebige einwertige Alkohole vorliegen. Bevorzugt werden jedoch solche Ausgangsmaterialien, in denen die Alkoholkomponente bis zu 5 Kohlenstoffatome enthält, wobei insbesondere der Kohlenwasserstoffrest des Alkohols Methyl, Äthyl, n-Propyl, Isopropyl, n-Butyl, sec.-Butyl und/oder tert.-Butyl ist. Die besonders bevorzugten Carbamate leiten sich vom tert.-Butyl-Alkohol oder Methyl-Alkohol ab, wobei den Methylcarbamaten nochmals eine Vorzugsstellung zukommt.

Es kann zweckmässig sein, im Reaktionsmedium ein organisches Lösungsmittel mitzuverwenden. Geeignete Lösungsmittel sind beispielsweise halogenierte Kohlenwasserstoffe, wie Methylenchlorid oder Äthylendichlorid.

Die Carbamatspaltung wird weiterhin bevorzugt in inerter Atmosphäre durchgeführt, wobei Stickstoff ein geeignetes Mittel ist.

Zur Carbamatspaltung wird das Ausgangsmaterial mit dem wässrig-halogenwasserstoffsauren Medium behandelt, bis die hinreichende Spaltung der Carbamatgruppe eingetreten ist. Es kann zweckmässig sein, das Reaktionsgemisch für einen Zeitraum von 6 oder mehr Stunden am Rückfluss zu erhitzen, um die vollständige Carbamatspaltung sicherzustellen. Es wurde jedoch weiterhin gefunden, dass die Reaktion — möglicherweise aufgrund verbesserter Löslichkeitsfaktoren — schneller abläuft, wenn kurzkettige organische Säuren dem Reaktionsgemisch beigegeben werden. Die bevorzugte kurzkettige organische Säure ist Essigsäure, die insbesondere als Eisessig zugesetzt wird, da die relative Säurestärke des Reaktionsgemisches derart auf die Reaktion einwirkt, dass der gewünschte Reaktionsablauf um so vollständiger ist, je höher die Säurestärke ist.

Bedeutungsvoll für die Carbamatesterspaltung ist auch die Beschaffenheit der Alkoholkomponente in der Carbamatgruppierung. In der Reihe tert.-Butyl- zu Methyl- zu Äthyl- zu Propyl- und zu den restlichen Butyl-Carbamaten besteht eine abnehmende Neigung zur Carbamatspaltung. Es ist überraschend, dass die zwei ungleichen Carbamate des Methanols und des tert.-Butanols die am leichtesten zu spaltenden Ausgangsverbindungen sind. Der tert.-Butylester kann sogar mit relativ schwachen Säuren, wie Essigsäure, Oxalsäure oder einfach Salzsäure, gespalten werden. Demgegenüber fordert der Methylester die zuvor geschilderten stark-sauren Hydrolysebedingungen.

Es wurde weiterhin gefunden, dass erfindungsgemäss bei der Verwendung von Bromwasserstoffsäure die Mitverwendung von Bromwasserstoffgas im Reaktionsgemisch nicht unbedingt erforderlich ist, während bei der Verwendung von Salzsäure ohne zusätzliche Sättigung mit Chlorwasserstoffgas schärfere Bedingungen eingesetzt werden müssen. Im allgemeinen gilt, dass die Reaktion im Bereich von 10°C bis 100°C oder darüber durchgeführt werden kann und dabei mit zunehmender Temperatur begünstigt wird.

Als Reaktionsprodukte der Spaltung entstehen in Abhängigkeit vom eingesetzten Ausgangsmaterial das halogenwasserstoffsaure Salz des 3-Oxo-pregn-4-en-20-amins und/oder unmittelbar das 3,20-Dioxo-pregn-4-en. Diese zuletzt genannte Verbindung entsteht offenbar durch spontane Umlagerung des aus der Hydrolyse des Esters von 3-Oxo-pregna-4,17-(20)-dien-20-carbaminsäure entstehenden Reaktionsproduktes. Das zunächst als beispielsweise Hydrochlorid anfallende Steroid-20-Amin kann in an sich bekannter Weise durch Behandlung mit einer starken Base zum freien Amin umgewandelt werden.

Es war nicht vorherzusehen, dass eine selektive Spaltung der Carbamatestergruppierung unter den erfindungsgemässen Bedingungen möglich ist, ohne dass im beträchtlichen Ausmass weitere Angriffe auf das Steroid-Ringgerüst stattfindet. So wurde beispielsweise gefunden, dass strukturgemäss nahe verwandte Verbindungen, die jedoch zusätzlich im A-Ring des Steroidsystems eine Doppelbindung in 1-2-Stellung aufweisen, gegenüber dem halogenwasserstoffsauren Reaktionsmedium nicht stabil sind. Überraschenderweise sind jedoch die erfindungsgemäss eingesetzten Systeme, die eine Doppelbindung in 4-5-Stellung und ggf. eine zusätzliche Doppelbindung in 17-20-Stellung aufweisen, unter den Bedingungen der halogenwasserstoffsauren Hydrolyse stabil. Die zuvor erwähnten Verbindungen mit einer zusätzlichen Doppelbindung in 1-2-Stellung des A-Rings unterliegen bei den für die Carbamatspaltung benötigten scharfen Bedingungen im halogenwasserstoffsauren Medium einer Ringhalogenierung und zusätzlich einer Aromatisierung der Ringstruktur.

Auch der Stand der Technik gab auf das erfindungsgemässe Verfahren und seine Möglichkeiten keinen Hinweis. Aus dem umfangreichen Stand der Technik zur Gewinnung von Progesteron und seinen Analogen sei verwiesen auf Canadian Journal of Chemistry, Band 34 (1956), S. 982-990; Helvetica Chimica Acta, Band XXXII, Teil VI (1949), Nr. 255, S. 1922-1933; a.a.O. Band XXXII, Teil V (1949), Nr. 233, S. 1764-1769. Verwiesen wird weiterhin auf

JACS, Band LXX (1948), Nr. 3, S. 887-892 sowie Helvetica Chimica Acta, Band XXXII, Teil V (1949), Nr. 232, S. 1758-1763 und schliesslich auf die US-PS 3 519 658.

*Beispiel*

Durch Umsetzung von 5,0 g 3-Oxo-pregn-4-en--20-methylcarbamat in 80 ml konzentrierter Salzsäure, die zuvor mit wasserfreiem Chlorwasserstoffgas gesättigt worden ist, wird das Hydrochlorid des 20-Amino-3-oxo-pregn-4-en erhalten. Das Reaktionsgemisch wird hierzu für den Zeitraum von mindestens 6 Stunden unter Stickstoff am Rückfluss gekocht. Die Säure wird dann unter vermindertem Druck abgezogen, der Rückstand wird in Methylenchlorid aufgenommen. Die Methylenchloridlösung wird 2mal mit gesättigter Salzlösung gewaschen und über Magnesiumsulfat getrocknet. Anschliessend wird das Lösungsmittel zur Trockne abgezogen, es fällt das Hydrochlorid des 20-Amino-3-oxo--pregn-4-en an.

Bei gleicher Reaktionsführung kann die Ausbeute an gewünschtem Reaktionsprodukt dadurch verbessert werden, dass 80 ml Eisessig dem Reaktionsgemisch zu Beginn zugegeben werden. Die hierbei erhaltene Gesamtausbeute liegt über 90% der Theorie, es bleibt nur wenig unumgesetztes Carbamat zurück. Wird demgegenüber im Reaktionssystem die Sättigung mit wasserfreiem Chlorwasserstoff fortgelassen, so muss die Zeitdauer der Hydrolysenreaktion verlängert werden, um vergleichbare Ausbeuten zu erhalten.

Wird der gleiche Reaktionsansatz wiederholt, dabei jedoch mit konzentrierter Bromwasserstoffsäure ohne Zusatz von wasserfreiem Bromwasserstoffgas und ohne Zusatz von Essigsäure gearbeitet, so wird das Reaktionsprodukt in vergleichbar guter Ausbeute erhalten.

Ebenfalls vergleichbare Ergebnisse erhält man bei der Verwendung des tert.-Butyl-Carbamatesters als Steroidausgangsmaterial. Das Methylcarbamat des 3-Oxo-pregna-4,17(20)-dien wird durch die beschriebene saure Hydrolyse zum Progesteron umgewandelt.

## Patentansprüche

1. Verfahren zur selektiven hydrolytischen Spaltung von Steroid-20-Carbamaten aus der Gruppe 3-Oxo-pregn-4-en-20-carbamat und/oder 3-Oxo--pregna-4,17(20)-dien-20-carbamat, dadurch gekennzeichnet, dass man zur Spaltung der Carbamatgruppe in 20-Stellung das Ausgangsmaterial der Behandlung mit einer Halogenwasserstoffsäure unterwirft.

2. Verfahren nach Anspruch 1, dadurch gekennzeichnet, dass man mit wässrig-konzentrierten Halogenwasserstoffsäuren arbeitet.

3. Verfahren nach Ansprüchen 1 und 2, dadurch gekennzeichnet, dass man zur Spaltung wässrig-konzentrierte Halogenwasserstoffsäuren einsetzt, die zusätzlich mit gasförmigem Halogenwasserstoff gesättigt sind.

4. Verfahren nach Ansprüchen 1 bis 3, dadurch gekennzeichnet, dass man als Reaktionsmedium konzentrierte Salzsäure einsetzt, die mit gasförmigem HCl gesättigt worden ist, wobei gewünschtenfalls auch ein entsprechendes wässriges Bromwasserstoffmedium eingesetzt werden kann.

5. Verfahren nach Ansprüchen 1 bis 4, dadurch gekennzeichnet, dass mit einem Reaktionsmedium gearbeitet wird, dem zusätzlich eine kurzkettige Carbonsäure, insbesondere Eisessig, zugesetzt worden ist.

6. Verfahren nach Ansprüchen 1 bis 5, dadurch gekennzeichnet, dass Steroid-20-Carbamate als Ausgangsmaterial eingesetzt werden, die Alkohole mit 1- bis 5 C-Atomen im Carbamatesterrest enthalten.

7. Verfahren nach Ansprüchen 1 bis 6, dadurch gekennzeichnet, dass im Temperaturbereich von 10°C bis 100°C und vorzugsweise bei einer Reaktionszeit bis zu 6 Stunden oder mehr gearbeitet wird.

## Claims

1. A process for the selective hydrolytic splitting of steroid-20-carbamates from the group of 3-oxo--pregn-4-ene-20-carbamate and/or 3-oxo-pregna--4,17(20)-diene-20-carbamate, characterized in that the starting material is subjected to the treatment with a hydrogen halide acid in order to decompose the carbamate group in the 20-position.

2. The process according to claim 1, characterized in that aqueous concentrated hydrogen halide acids are used.

3. The process according to claims 1 and 2, characterized in that aqueous concentrated hydrogen halide acids that have additionally been saturated with the gaseous hydrogen halide are used for the decomposition.

4. The process according to claims 1 to 3, characterized in that concentrated hydrochloric acid having been saturated with gaseous HCl is used as the reaction medium, in which process also a respective aqueous hydrogen bromide medium may be used if desired.

5. The process according to claims 1 to 4, characterized in that a reaction medium is used to which a short-chain carboxylic acid, more particularly glacial acetic acid, has been added as an additional component.

6. The process according to claims 1 to 5, characterized in that steroid-20-carbamates containing alcohols having from 1 to 5 carbon atoms in the carbamate ester residue are used as the starting materials.

7. The process according to claims 1 to 6, characterized in that it is effected in the temperature range of from 10°C to 100°C and preferably using a reaction time of up to 6 hours or more.

## Revendications

1. Procédé pour la dissociation sélective par hydrolyse de stéroïde-20-carbamates du groupe de 3-oxo-prégna-4-ène-20-carbamates et/ou 3-oxo--prégna-4,17(20)-diène-20-carbamates, caractérisé en ce que, pour la dissociation du groupe carbamate en position 20, on soumet le produit de départ au traitement par un acide halohydrique.

2. Procédé selon la revendication 1, caractérisé en ce que l'on opère avec des acides halohydriques concentrés aqueux.

3. Procédé selon les revendications 1 et 2, caractérisé en ce que l'on utilise pour la dissociation des acides halohydriques concentrés aqueux qui sont en outre saturés par l'halogénure d'hydrogène gazeux.

4. Procédé selon les revendications 1 à 3, caractérisé en ce que l'on utilise comme milieu de réaction l'acide chlorhydrique concentré qui a été saturé par HCl gazeux, et l'on peut également utiliser aussi un milieu d'acide bromhydrique aqueux correspondant.

5. Procédé selon les revendications 1 à 4, caractérisé en ce que l'on opère avec un milieu de réaction auquel on a ajouté un acide carboxylique à chaîne courte, en particulier l'acide acétique.

6. Procédé selon les revendications 1 à 5, caractérisé en ce que l'on utilise comme produit de départ des stéroïde-20-carbamates qui contiennent dans le reste ester carbamique des alcools en $C_1$-$C_5$.

7. Procédé selon les revendications 1 à 6, caractérisé en ce que l'on opère dans un intervalle de température de 10°C à 100°C et de préférence avec une durée de réaction allant jusqu'à 6 h ou plus.